# EUROPEAN PATENT SPECIFICATION

(11) **EP 0 931 558 B1**
(45) Date of publication and mention of the grant of the patent: **29.10.2003**
(21) Application number: 99101045.5
(22) Date of filing: 22.01.1999
(51) Int. Cl.: A61M 25/00

(54) **A catheter**
Katheter
Cathéter

(30) Priority: 23.01.1998 JP 2662198
(43) Date of publication of application: 28.07.1999
(73) Proprietor: ASAHI INTECC CO., LTD., Seto-shi, Aichi-ken (JP)
(72) Inventor: Miyata, Masahiko, Seto-shi, Aichi-ken (JP); Kato, Tomihisa, Seto-shi, Aichi-ken (JP)
(74) Representative: VOSSIUS & PARTNER

(56) References cited:
- EP-A- 0 102 685
- EP-A- 0 420 993
- WO-A-96/33763

## Description

The present invention relates to a catheter used for diagnosis or therapeutics.

A catheter used for diagnosis or therapeutics is guided by guide wire from the outside of the body into the blood vessel, the trachea, the gonaduct, the ureter, etc. to reach a part to be treated to perform diagnosis or therapeutics. Accordingly, said catheter should be flexible enough to bend freely in the blood vessel etc. and not to injure the wall of the blood vessel etc. while said catheter should have a good torque transmission property and said catheter should be rigid enough for the good torque transmission property.

Hitherto, to solve said antinomy, catheters consisting of a base part having enough rigidity to transmit the torque and an end part having flexibility to bend freely have been presented (TOKKAISho 60-31765, Japanese Patent No. 2523405).

In TOKKAISho 60-31765, the tube wall of the catheter has a two-layer structure consisting of an inner tube part and an outer tube part and when the flexibility of said inner tube part is set to be smaller than the flexibility of said outer tube part, the thickness of said inner tube part in said base part is set to be larger than the thickness of said inner tube part in said end part, and when the flexibility of said inner tube part is set to be larger than the flexibility of said outer tube part, the thickness of said outer tube part in said base part is set to be larger than the thickness of said outer tube part in said end part to give said base part high rigidity for a good torque transmission property and said end part high flexibility to bend freely.

Further, in Japanese Patent No. 2523405, a catheter having a rigid base part and a flexible end part is provided wherein the glass transition temperature of the organic material of said base part is set to be higher than the glass transition temperature of the organic material of said flexible end part and further, a metal reinforcement is wound on the outside of said base part and still further, a synthetic resin coating is applied on said reinforcement to give said base part high rigidity.

Said catheter of TOKKAISho 60-31765 has faults in that the flexibility of said inner tube part is much different from the flexibility of said outer tube part, resulting in poor interlaminate strength between said inner tube part and said outer tube part and said catheter is easily folded by stress concentration.

Further, said catheter of Japanese Patent No. 2523405 has a fault in that the unevenness of the metal reinforcement appears on the surface of synthetic resin coating.

Another catheter is disclosed in WO-A-9633763. This document relates to catheter section comprising an elongate tubular member having a proximal end and a distal end and a passageway defining an inner lumen extending between those ends. The wall of the tubular member has a polylayer structure with at least three layers consisting of a base part and an end part. Said base part is reinforced by a metal reinforcement arranged on the outside of the inner layer. The glass transition temperature of said polylayer structure becomes gradually lower from the inside to the outside.

Accordingly, an object of the present invention is to present a catheter consisting of a base part having enough rigidity for a good torque transmission property and an end part having enough flexibility to bend freely but also allowing safe diagnostic and therapeutic procedures without injuring a blood vessel.

To attain this object, the present invention provides a catheter as defined in claim 1. In particular, this catheter comprises a tube wall having a polylayer structure with at least three layers.

Said catheter consists of a base part and an end part, and said base part is reinforced by a metal reinforcement arranged on the outside of the inner layer to give said base part enough rigidity.

The glass transition temperature of said polylayer becomes gradually lower from the inside to the outside and the glass temperature of the outer layer is equal to or lower than the bodily temperature.

The material of said outer layer is in the rubbery state at the bodily temperature when said catheter is inserted into the body. The bending modulus of elasticity of said material is more than 10000 N/cm² (1000 kgf/cm²) at a temperature close to the bodily temperature, the material of said inner layer keeps the glassy state at the bodily temperature when said catheter is inserted into the body, and the bending modulus of elasticity of said material is more than 25000 N/cm² (2500 kgf/cm²) at 23°C.

Further, it is preferable that said catheter has a three layer structure consisting of an inner layer, a middle layer, and an outer layer and said metal reinforcement arranged on the outside of the inner layer is covered with said middle layer and said middle layer and outer layer consist of the same material.

Still further, it is preferable that 150000 N/cm² (15000 kgf/cm²) ≧ G₁ ≧ 80000 N/cm² (8000 kgf/cm²) and 35000 N/cm² (3500 kgf/cm²) ≧ G³ ≧ 10000 N/cm² (1000 kgf/cm²) wherein G₁ is the bending modulus of elasticity of said inner layer and G₃ is the bending modulus of elasticity of said outer layer.

Still further, it is preferable that the thickness of the inner layer of said base part is larger than the thickness of the inner layer of said end part.

The invention will be described with reference to preferred embodiments and the drawings, in which:
Figure 1 is the enlarged partial cross sectional view of a catheter showing the end part thereof.
Figure 2 is the whole side view of said catheter.
Figure 3 is the cross sectional view of the boundary part between the base part and the end part.
Figure 4 is a perspective view to illustrate the manufacturing process of a catheter.
Figure 5 is the enlarged cross sectional view of the boundary part between the base part and the end part.

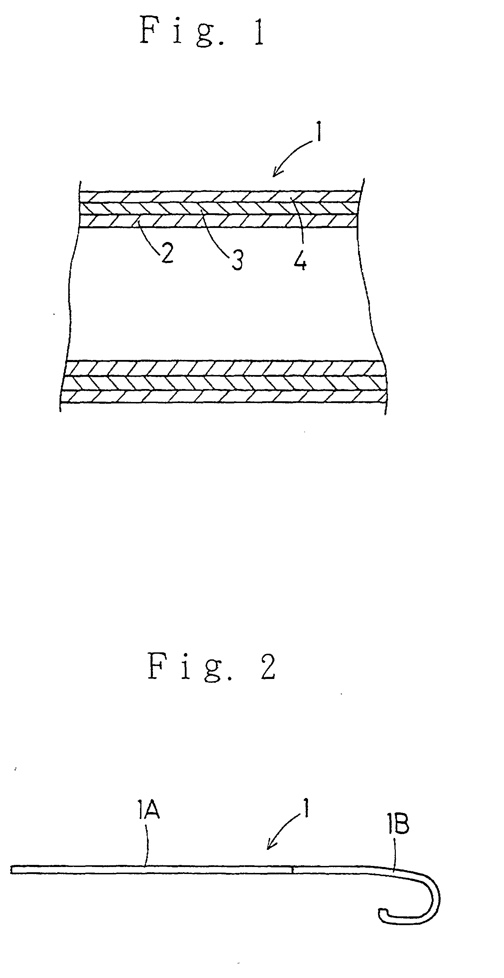

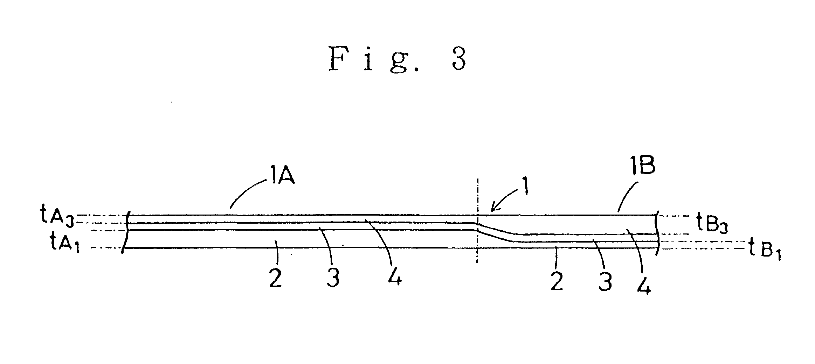

Figure 1 to Figure 3 relate to an embodiment of the present invention. A catheter (1) shown in Figures has a tube wall having a three-layer structure which consists of an inner layer (2), a middle layer (3) and an outer layer (4) and assuming the bending modulus of elasticity of said inner layer (2) at a temperature close to the bodily temperature is G₁, the bending modulus of elasticity of said middle layer (3) at a temperature close to the bodily temperature is G₂, and the bending modulus of elasticity of said outer layer (4) at a temperature close to the bodily temperature is G₃, then 150000 N/cm² (15000 kgf/cm²) ≧ G₁ ≧ G₂ ≧ G₃ ≧ 10000 N/cm² (1000 kgf/cm²), preferably 150000 N/cm² (15000 kgf/cm²) ≧ G₁ ≧ 80000 N/cm² (8000 kgf/cm²), 60000 N/cm² (6000 kgf/cm²) ≧ G₂ ≧ 40000 N/cm² (4000 kgf/cm²), 35000 N/cm² (3500 kgf/cm²) ≧ G₃ ≧ 10000 N/cm² (1000 kgf/cm²) and the material of said inner layer (2) keeps the glassy state at the bodily temperature when said catheter (1) is inserted into the body and the material of said outer layer (4) is in the rubbery state at the bodily temperature when said catheter (1) is inserted into the body. Accordingly, the glass transition temperature Tg₁ of said inner layer (2) is set to be higher than the bodily temperature Tₒ (Tg₁ >Tₒ)and the glass transition temperature Tg₃ of said outer layer (4) is set to be equal to or lower than the bodily temperature Tₒ (Tg₃ ≦ Tₒ). Wherein Tₒ is, for example, in the range between 35 to 37°C but may reach 40°C when the body has hyperthermia.

Further, the material of each layer of said catheter (1) is an incomplete amorphous material, and the crystallinity of said outer layer is equal to or lower than 30% at the bodily temperature Tₒ.

The material of said catheter is, for example, urethane elastomers, styrene elastomers, ester elastomers, olefin elastomers, amido elastomers, fluoroelastomers or mixtures of two or more kinds of these elastormers. A filler having radiopacity such as barium sulfate and the like is preferably added to said elastomers in an amount between 30 to 50% by weight so that a catheter can be detected by radiography.

As shown in Figure 2, said catheter (1) consists of a base part (1A) and an end part (1B), and generally it is preferable that said base part (1A) has high rigidity and said end part (1B) has flexibility.

To give said base part (1A) high rigidity, and said end part (1B) flexibility, the thickness tA₁ of said inner layer (2) of said base part (1A) is set to be larger than the thickness tB₁ of said inner layer (2) of said end part (1B), and the thickness tA₃ of said outer layer (4) of said base part (1A) is set to be less than the thickness tB₃ of said inner layer (2) of said end part (1B). Concretely, in said base part (1A), 0.18 mm ≦ tA₁ ≦ 0.19 mm, 0.03 mm ≦ tA₃ ≦ 0.05 mm, tA₁>tA₃, and in said end part (1B), 0.07 mm≦ tB₁ ≦ 0.09 mm, 0.14 mm ≦ tB₃ ≦ 0.15 mm, tB₁ < tB₃ and tA₁ > tB₁, tA₃ < tB₃.

Total thickness of the tube wall of said catheter (1) is generally 0.6 to 0.7 mm so that the thickness tA₂ of said middle layer (3) of said base part (1A) is t - tA ₁ - tA₃ and the thickness tB₂ of said middle layer (3) of said end part (1B) is t - tB₁ - tB₃ (tA₂ = t - tA₁ - tA₃, tB₂ = t - tB₁ - tB₃).

When said catheter (1) of this embodiment is inserted into the body and effected by the bodily temperature, the bending modulus of elasticity G ₃ of said outer layer (4) is set to be low, such as 35000 N/cm² (3500 kgf/cm²) ≧ G₃ ≧ 10000 N/cm² (1000 kgf/cm²), and said outer layer (4) is set to be in the rubbery state, so that said catheter (1) has enough flexibility to bend easily following the curve shape of the blood vessel and said outer layer (4) can follow a curve having a small curvature radius without cracking or breaking.

Further, when the tube is molded by extrusion using a rigid material, sinks may be formed on the surface of the tube. However, the inside layer of said rigid material is covered with the outside layer of a soft material having good fluidity filling up said sinks of the inside layer to obtain a catheter having a smooth surface without unevenness. If a catheter has an uneven surface, it is feared that the thrombus adheres to said catheter resulting in the difficulty of inserting said catheter into the blood vessel.

As above described, said outer layer of the catheter is so soft that safety in diagnosis or therapeutic is guaranteed without injuring the blood vessel and the like.

Further, as said inner layer (2) of said catheter (1) has a high bending modulus of elasticity G₁ such as 150000 N/cm² (15000 kgf/cm²) ≧ G₁ ≧ 80000 N/cm² (8000 kgf/cm²) and the glass temperature of said inner layer (2) is set to be higher than the bodily temperature, said catheter (1) has an enough torque transmission property when said catheter (1) is inserted into the body and easy operation for diagnosis or therapeutic is guaranteed.

Still further, as the bending modulus of elasticity becomes gradually lower from the inner layer to the outer layer, the difference of the mechanical property between a pair of layers attaching to each other may become small, resulting in high interlaminate strength and folding by stress concentration is effectively avoided.

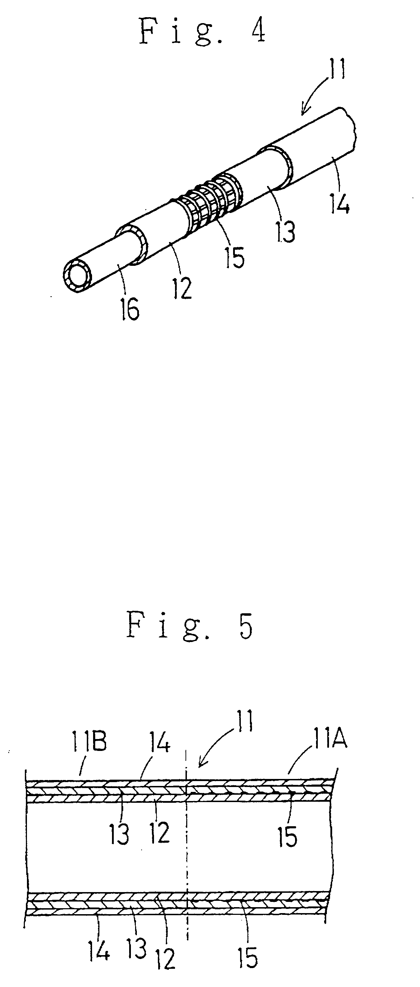

Figure 4 illustrates the manufacturing process of a catheter. The outside of a core bar (16) is covered with a melted material or a solution of a material by coating or extrusion to form an inner layer (12) by cooling or drying and then a tubular metal net is wound on said inner layer (12) in a base part (11A) as a metal reinforcement (15). The same material as used in the prior embodiment is used in this embodiment.

Said metal reinforcement (15) is covered with a middle layer (13) and further said middle layer (13) is covered with an outer layer (14). Said middle layer (13) and said outer layer (14) may be formed by coating or extrusion using the same material as used in the prior embodiment. Preferably, the same material is used for said middle layer (13) and said outer layer (14) to obtain higher interlaminate strength.

As shown in Figure 5, a catheter (11) having three layer structure consisting of said inner layer (12), and middle layer (13) and said outer layer (14) is manufactured by the process as above described. Said base part (11A) has high rigidity by inserting said metal reinforcement (15) between said inner layer (12) and said middle layer (13) to obtain a good torque transmission property. Further, said catheter (11) has a smooth surface without unevenness since unevenness of the surface of said middle layer caused by unevenness of said metal reinforcement is filled by said outer layer (14), and said end part (11B) has enough flexibility since no metal reinforcement is inserted in the end part (11B).

The catheter of the present invention has a polylayer structure with at least three layers and in the case where the mechanical property changes gradually from inside layer to outside layer to obtain the minimum difference of the mechanical property between a pair of layers attaching to each other, high interlaminate strength is guaranteed and stress concentration is avoided.

## Claims

1. A catheter (1, 11) whose tube wall has a polylayers structure with at least three layers consisting of a base part (1A, 11A) and an end part (1B, 11B), said base part (1A, 11A) is reinforced by a metal reinforcement (15) arranged on the outside of an inner layer (2, 12) wherein the glass transition temperature T_{g1} of said polylayer structure becomes gradually lower from the inside to the outside the outer layer has a crystallinity equal to or lower than 30% and is set to be in the rubbery state at the bodily temperature T₀ so that the glass transition temperature of the outer layer (4, 14) is set to be equal to or lower than the bodily temperature T₀ and the inner layer is set to be in the glass state at the bodily temperature T₀ so that the glass transition temperature of the inner layer is set to be higher than the bodily temperature T₀.

2. A catheter (1, 11) of claim 1, wherein said catheter (1, 11) has a three-layers structure consisting of an inner layer (2, 12), a middle layer (3, 13), and an outer layer (4, 14) and said reinforcement is covered with the middle layer (3, 13) and further the middle layer (3, 13) and the outer layer (4, 14) are made of the same material.

3. A catheter (1, 11) of claim 1 or 2, wherein the thickness of the inner layer (2, 12) of said base part is (1A, 11A) is larger than the thickness of the inner layer (2, 12) of said end part (1B, 11B).

4. A catheter (1,11) according to claim 1, 2 or 3 wherein a filler having radiopacity is added in at least one layer.

## Patentansprüche

1. Katheter (1,11), dessen Röhrenwand eine Mehrschichtstruktur mit mindestens drei Schichten aufweist, der aus einem Basisteil (1A,11A) und einem Endteil (1B,11B) besteht, wobei das Basisteil (1A,11A) durch eine Metallverstärkung (15) verstärkt ist, die auf der Außenseite einer inneren Schicht (2,12) angeordnet ist, wobei die Glasumwandlungstemperatur Tg₁ der Mehrschichtstruktur von der Innenseite zur Außenseite allmählich niedriger wird, die äußere Schicht eine Kristallinität aufweist, die gleich oder niedriger als 30% ist und so eingestellt wird, daß sie sich bei der Körpertemperatur Tₒ im gummiartigen Zustand befindet, so daß die Glasumwandlungstemperatur der äußeren Schicht (4,14) so eingestellt wird, daß sie gleich oder niedriger als die Körpertemperatur Tₒ ist, und die innere Schicht so eingestellt wird, daß sie sich bei der Körpertemperatur Tₒ im Glaszustand befindet, so daß die Glasumwandlungstemperatur der inneren Schicht so eingestellt wird, daß sie höher als die Körpertemperatur Tₒ ist.

2. Katheter (1,11) nach Anspruch 1, wobei der Katheter (1,11) eine Dreischichtstruktur aufweist, die aus einer inneren Schicht (2,12), einer mittleren Schicht (3,13) und einer äußeren Schicht (4,14) besteht, und die Verstärkung mit der mittleren Schicht (3,13) bedeckt ist, und ferner die mittlere Schicht (3,13) und die äußere Schicht (4,14) aus demselben Material bestehen.

3. Katheter (1,11) nach Anspruch 1 oder 2, wobei die Dicke der inneren Schicht (2,12) des Basisteils (1A,11A) größer als die Dicke der inneren Schicht (2,12) des Endteils (1B,11B) ist.

4. Katheter (1, 11) nach Anspruch 1, 2 oder 3, wobei ein Füllstoff, der eine Röntgenundurchlässigkeit aufweist, in mindestens einer Schicht hinzugegeben wird.

## Revendications

1. Un catheter (1,11) dont la paroi tubulaire a une structure multicouches avec au moins trois couches consistant en une partie de base (1A, 11A) et une partie finale (1B, 11B), ladite partie de base (1A, 11A) étant renforcée par un renforcement en métal (15) disposé sur l'extérieur d'une couche interne (2, 12), dans lequel la température de transition vitreuse Tg₁ de ladite structure multicouche diminue graduellement depuis l'intérieur vers l'extérieur, la couche externe a une cristallinité égale ou inférieure à 30% et est ajusté pour être à l'état caoutchouteux à la température corporelle Tₒ de telle manière que la température de transition vitreuse de la couche externe (4, 14) soit ajustée pour être égale ou inférieure à la température corporelle Tₒ et la couche interne est ajustée pour être à l'état vitreux à la température corporelle Tₒ de telle manière que la température de transition vitreuse de la couche interne soit ajustée pour être plus élevée que la température corporelle Tₒ.

2. Un cathéter (1, 11) selon la revendication 1, dans lequel ledit catheter (1, 11) a une structure tri-couches consistant en une couche interne (2, 12), une couche médiane (3, 13), et une couche externe (4, 14) et ledit renforcement est couvert par la couche médiane (3, 13) et par ailleurs la couche médiane (3, 13) et la couche externe (4, 14) sont réalisées dans le même matériau.

3. Un catheter (1, 11) selon l'une des revendications 1 ou 2, dans lequel l'épaisseur de la couche interne (2, 12) de la partie de base (1A, 11A) est supérieure à l'épaisseur de la couche interne (2, 12) de la partie finale (1B, 11B).

4. Un catheter (1, 11) selon l'une des revendications 1, 2 ou 3 dans lequel une charge présentant une opacité aux radiations est ajoutée dans au moins l'une des couches.
